# EUROPÄISCHE PATENTSCHRIFT

(11) **EP 2 293 834 B1**
(45) Veröffentlichungstag und Bekanntmachung des Hinweises auf die Patenterteilung: **22.08.2018**
(21) Anmeldenummer: 09761389.7
(22) Anmeldetag: 26.05.2009
(51) Int. Cl.: A61M 15/08, B05B 11/00

(54) **FLUIDAUSTRAGKOPF**
FLUID DISCHARGE HEAD
TÊTE DE DÉCHARGE DE FLUIDE

(30) Priorität: 10.06.2008 DE 102008027599
(43) Veröffentlichungstag der Anmeldung: 16.03.2011
(73) Patentinhaber: Silgan Dispensing Systems Hemer GmbH, 58675 Hemer (DE)
(72) Erfinder: WELP, Gisbert, 59846 Sundern (DE)
(74) Vertreter: Henseler, Daniela
(86) Internationale Anmeldenummer: PCT/EP2009/003722
(87) Internationale Veröffentlichungsnummer: WO 2009/149826

(56) Entgegenhaltungen:
- EP-A- 0 901 836
- WO-A1-03/026803
- DE-A1- 3 315 334
- DE-A1- 19 622 124
- DE-A1-102004 050 679
- DE-U1- 29 811 242

## Beschreibung

Die Erfindung betrifft einen Fluidaustragkopf nach dem Oberbegriff des Anspruchs 1.

Ein gattungsgemäßer Fluidaustragkopf ist bekannt aus WO 03/026803 A1.

Aus WO 2007/009617 A1 ist ein Fluidaustragkopf mit einem eine Austragöffnung aufweisenden Austragstutzen, der eine Innenhülse aufnimmt, bekannt. In der Innenhülse ist ein Innenkörper angeordnet, der einen Auslasskanal begrenzt und ein Verbindungsglied zur Verbindung mit dem Gegenstück einer Austragvorrichtung aufweist. Die Innenhülse weist stirnseitig benachbart zur Austragöffnung eine Dichtfläche auf, gegen die ein am Innenkörper befindlicher, den Auslasskanal verschließender Ventilstopfen federvorgespannt ist. In den Austragkopf ist damit ein Ventil integriert, bei dem der Ventilverschluss durch eine Relativbewegung bei Betätigung durch den Anwender realisiert wird. Fluidaustragkopf und Austragvorrichtung sind dazu axial gegeneinander bewegbar. Bei einer Freigabe der Betätigungskraft kehren durch eine Feder die Einheiten in entgegengesetzter Richtung zur Ausgangsstellung zurück. Ferner ist eine Abziehsicherung vorgesehen, die sicherstellt, dass Fluidaustragkopf und Austragvorrichtung nicht entgegen der Betätigungsrichtung voneinander gelöst werden.

Die Abziehsicherung weist dazu einen ringförmigen Schnappnocken und einen Mantel an einem Austragstutzen des Fluidaustragkopfes auf. Nachteilig ist, dass die Abziehsicherung vollständig innerhalb eines Gehäuses des Fluidaustragkopfes liegt. Das Gehäuse besitzt einen weit vorstehenden Kappenmantel, der die Handlichkeit des aus Fluidaustragkopf und Austragvorrichtung zusammengesetzten Spenders verschlechtert.

Aufgabe der Erfindung ist es daher, einen Fluidaustragkopf zu schaffen, der eine verbesserte Handhabung erlaubt.

Diese Aufgabe wird durch die Merkmale des Anspruchs 1 gelöst.

Hierdurch wird ein Fluidaustragkopf geschaffen, bei dem eine zusätzliche, zweite Schürze die Führung des Fluidaustragkopfes gegenüber der Austragvorrichtung verbessert, und zwar in Verbindung mit einer Abziehsicherung. Die Abziehsicherung ist damit integriert in ein Führungselement, das die axiale Bewegung von Fluidaustragkopf und Austragvorrichtung gegeneinander bei einer Betätigung des aus ihnen zusammengesetzten Spenders lenkt.

Weitere Ausgestaltungen der Erfindung sind der nachfolgenden Beschreibung und den Unteransprüchen zu entnehmen.

Die Erfindung wird nachstehend anhand des in den beigefügten Abbildungen dargestellten Ausführungsbeispiels näher erläutert.
Fig. 1 zeigt schematisch im Schnitt einen Fluidaustragkopf mit einem Teilstück einer Austragvorrichtung,

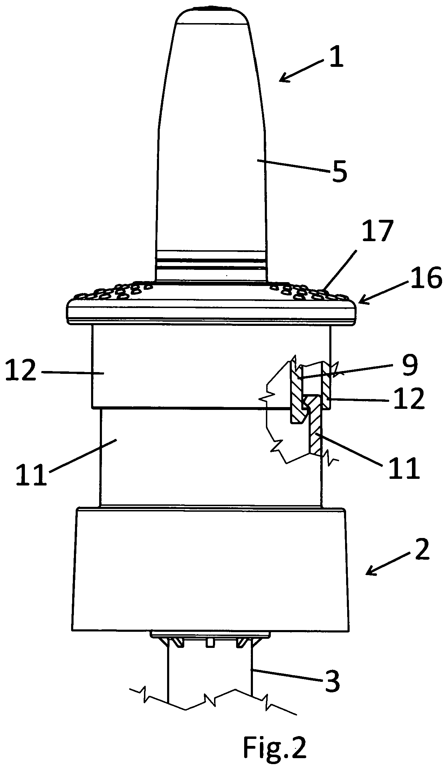

Fig. 2 zeigt die beiden Einheiten gemäß Fig. 1 in einer Ausbruchsdarstellung.

Die Erfindung betrifft einen Fluidaustragkopf 1 zur Verwendung mit einer Austragvorrichtung 2, wobei die Austragvorrichtung 2 einen nicht dargestellten Mediumspeicher für Fluid umfasst, in dem das Medium unter Druck gestellt ist oder aus dem das Medium über eine Medienpumpe 3, insbesondere eine Schubkolbenpumpe, ausgetragen wird. Die Austragvorrichtung 2 weist vorzugsweise ein Gegenstück 4 auf, an dem der Fluidaustragkopf 1 anbringbar ist.

Die Austragvorrichtung 2 mit aufgesetztem Fluidaustragkopf 1 bilden einen Spender für insbesondere flüssige Medien.

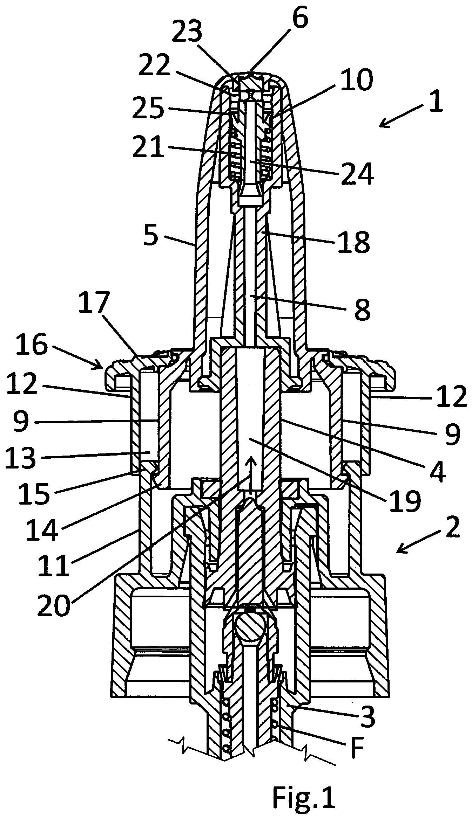

Der Fluidaustragkopf 1 und die Austragvorrichtung 2 sind zur Austragsbetätigung unter Verkürzung des Spenders axial gegeneinander bewegbar. Bei Freigabe einer Betätigungskraft kehren sie durch eine Feder F in entgegengesetzter Richtung zur Ausgangsstellung gemäß Fig. 1 zurück.

Der Fluidaustragkopf 1 weist einen auf die Austragvorrichtung 2 aufsetzbaren Austragstutzen 5 auf, der eine Medienführung 8 in Form eines Medienkanals und einen Medienauslass mit einer Austragöffnung 6 am Ende einer Medienführung 8 aufnimmt. Im Bereich des Medienauslasses ist ein bewegliches, in eine Öffnungsstellung überführbares Verschlusselement 10 vorgesehen, das als federvorgespannter Ventilkörper ausgebildet ist.

Der Austragstutzen 5 weist eine Schürze 9 auf zur Bildung einer Gleitführung mit einem Abschnitt 11 an der Austragvorrichtung 2. Dieser Schürze 9 des Austragsstutzens 5 ist eine zweite, zusätzliche Schürze 12 zugeordnet zur Ausbildung einer Ringbuchse 13. Die Ringbuchse 13 ist in Axialrichtung längsverschieblich lagerbar auf einem als Ringschaft ausgebildeter Abschnitt 11 der Austragvorrichtung 2. Die zweite, zusätzliche Schürze 12 ist vorzugsweise radial federnd ausgebildet. Dazu kann die zweite, zusätzliche Schürze 12 kürzer ausgebildet sein als die andere Schürze 9. Die zweite, zusätzliche Schürze ist vorzugsweise derart kürzer ausgebildet ist, dass eine radiale Einschnapprampe zwischen den beiden Schürzen 9, 12 entsteht.

Die zweite, zusätzliche Schürze 12 ist eine äußere Schürze 12, während die andere Schürze 9 eine innere Schürze 9 ist. In die Ringbuchse 13 ragt ein Nocken 14 für eine Abziehsicherung. Der Nocken 14 ist vorzugsweise an der inneren Schürze 9 als Nockenrand vorgesehen. Dem Nocken 14 ist ein Gegennocken 15 am Abschnitt 11 ausgebildet.

Bevorzugt ist ferner, dass an dem Stutzen 5 ein Aufsteckteil 16 mit einer Grifffläche 17 vorgesehen ist. An diesem Aufsteckteil 16 ist dann vorzugsweise die zweite, zusätzliche Schürze 12 ausgebildet. Das Aufsteckteil 16 kann aus einem anderen Material als der Austragstutzen 5 gefertigt sein. Hierdurch besteht die Möglichkeit, die beiden Schürzen 9, 12 aus unterschiedlichen Materialen zu fertigen. Insbesondere kann die zweite, zusätzliche Schürze 12 aus einem weicheren Kunststoff bestehen als die andere Schürze 9.

Der Austragstutzen 5 nimmt vorzugsweise eine Innenhülse 18 auf, die den Medienkanal 8 begrenzt, der an einen Austragabschnitt 19 der Medienführung 20 in Form von aneinander anschließenden und innerhalb des Fluidaustragkopfes 1 liegenden Kanalabschnitten und/oder Medienräumen anschliesst.

Die Innenhülse 18 ist an ihrem der Austragöffnung 6 zugewandten Ende topfförmig. Zum Verschließen der Austragöffnung 6 nimmt die Innenhülse 18 einen die Austragöffnung 6 selbsttätig schließenden federbelasteten Ventilkörper 10 in diesem topfförmigen Ende auf.

Der Ventilkörper 10 ist als zylindrischer Kolben ausgebildet, der mit einer Vorspannkraft durch eine Druckfeder 21 als federbelasteter Ventilkörper 10 die Austragöffnung 6 verschliesst. Der Ventilkörper 10 weist einen Zwischenventilteller 25 auf, der eine an den Medienkanal 8 angeschlossene Druckkammer 22 zu einem oberen Ventilsitz 23 des Ventilkörpers 10 abdichtet. Der Zwischenventilteller 25 dient ferner vorzugsweise zur Führung der Bewegung des Ventilkörpers 10.

Zum Öffnen des oberen Ventilsitzes 23 ist in der Druckkammer 22 ein Medienaustragdruck einstellbar, der höher ist als eine den Ventilkörper 10 zuhaltende Federkraft der Druckfeder 21. Die Fig. 1 zeigt eine durch den Ventilkörper 10 geschlossene Austragöffnung 6.

Der Ventilkörper 10 wird von Medium durchströmt, wozu der Ventilkörper 10 einen Durchlasskanal 24 aufweist, der den Medienkanal 8 mit der Druckkammer 22 verbindet.

Der Austragstutzen 5 besitzt hier die Form einer Nasenolive, um als Nasenadapter auf das Gegenstück 4 aufgesetzt werden zu können. Für andere Anwendungszwecke kann der Austragstutzen 5 andere Außenkonturen besitzen.

## Patentansprüche

1. Fluidaustragkopf (1) mit einem auf einer Austragvorrichtung (2) aufsetzbaren Austragstutzen (5), der eine Medienführung und einen Medienauslass am Ende der Medienführung aufnimmt, im Bereich des Medienauslasses ein bewegliches, in eine Öffnungsstellung überführbares Verschlusselement vorgesehen ist und zur Austragbetätigung der Austragstutzen (5) und die Austragvorrichtung (2) axial gegeneinander bewegbar sind, wozu der Austragstutzen (5) eine Schürze (9) aufweist zur Bildung einer Gleitführung mit einem Abschnitt (11) an der Austragvorrichtung (2), der Schürze (9) des Austragstutzens (5) eine zweite, zusätzliche äußere Schürze (12) zugeordnet ist zur Ausbildung einer Ringbuchse (13), die längsverschieblich lagerbar ist auf einem als Ringschaft ausgebildeten Abschnitt (11) der Austragvorrichtung (2), und die Ringbuchse (13) einen Nocken (14) für eine Abziehsicherung aufweist, **dadurch gekennzeichnet, dass** das Verschlusselement einen federbelasteten Ventilkörper (10) aufweist, der als zylindrischer Kolben ausgebildet ist, der in einem von einer einen Medienkanal (8) der Medienführung begrenzenden Innenhülse (18) des Austragstutzens (5) gebildeten topfförmigen Ende axial verschiebbar ist, und der Kolben einen Zwischenventilteller (25) aufweist, der eine an die Medienführung angeschlossene Druckkammer (22) zu einem oberen Ventilsitz (23) des Ventilkörpers abdichtet, die Medienführung (8) als Durchlasskanal (24) durch den Ventilkörper (10) sich erstreckt, so dass der Ventilkörper (10) von Medium durchströmt wird, wozu der Ventilkörper (10) den Durchlasskanal (24) aufweist, der den Medienkanal (8) mit der Druckkammer (22) verbindet.

2. Fluidaustragkopf nach Anspruch 1, **dadurch gekennzeichnet, dass** die zweite, zusätzliche Schürze (12) radial federnd ausgebildet ist.

3. Fluidaustragkopf nach Anspruch 1 oder 2, **dadurch gekennzeichnet, dass** die zweite, zusätzliche Schürze (12) derart kürzer ausgebildet ist als die andere Schürze (9), dass eine radiale Einschnapprampe vorgesehen ist.

4. Fluidaustragkopf nach einem der Ansprüche 1 bis 3, **dadurch gekennzeichnet, dass** zum Öffnen des oberen Ventilsitzes (23) in der Druckkammer (22) ein Medienaustragdruck einstellbar ist, der höher ist als eine den Ventilkörper (10) zuhaltende Federkraft.

5. Fluidaustragkopf nach einem der Ansprüche 1 bis 4, **dadurch gekennzeichnet, dass** an dem Stutzen (5) ein Aufsteckteil (16) mit einer Grifffläche (17) vorgesehen ist.

6. Fluidaustragkopf nach Anspruch 5, **dadurch gekennzeichnet, dass** an dem Aufsteckteil (16) die zweite, zusätzliche Schürze (12) ausgebildet ist, die aus einem anderen Material als der Austragstutzen (5) gefertigt ist.

7. Fluidaustragkopf nach Anspruch 6, **dadurch gekennzeichnet, dass** die zweite, zusätzliche Schürze (12) aus einem weicheren Kunststoff besteht als die andere Schürze (9).

## Claims

1. Fluid discharge head (1) having a discharge nozzle (5) which can be placed on a discharge device (2) and which holds a medium guide and a medium outlet at the end of the medium guide, in the region of the medium outlet a movable closure element that can be moved into an open position is provided and, for the purpose of actuating discharge, the discharge nozzle (5) and the discharge device (2) can be moved axially towards each other, for which purpose the discharge nozzle (5) has a skirt (9) to form a sliding guide with a section (11) on the discharge device (2), the skirt (9) of the discharge nozzle (5) is assigned a second, additional skirt (12) in order to form an annular bush (13) which can be mounted such that it can be displaced longitudinally on a section (11) of the discharge device (2) that is formed as an annular shaft, and the annular bush (13) having a cam (14) for an anti-removal safeguard, **characterized in that** the closure element has a spring-loaded valve body (10), which is formed as a cylindrical piston, which can be displaced axially in a pot-shaped end formed by an inner sleeve (18) of the discharge nozzle (5) limiting a medium duct (8) of the medium guide, and the piston has an intermediate valve plate (25) which seals off a pressure chamber (22) connected to the medium guide in relation to an upper valve seat (23) of the valve body, the medium guide (8) extending through the valve body (10) as a passage duct (24), so that medium flows through the valve body (10), for which purpose the valve body (10) has the passage duct (24) which connects the medium duct (8) to the pressure chamber (22).

2. Fluid discharge head according to claim 1, **characterized in that** the second, additional skirt (12) is designed to be radially springy.

3. Fluid discharge head according to claim 1 or 2, **characterized in that** the second, additional skirt (12) is formed so as to be shorter than the other skirt (9) in such a way that a radial snap-in ramp is provided.

4. Fluid discharge head according to one of claims 1 to 3, **characterized in that**, in order to open the upper valve seat (23), a medium discharge pressure which is higher than a spring force holding the valve body (10) closed can be set in the pressure chamber (22).

5. Fluid discharge head according to one of claims 1 to 4, **characterized in that** a push-on part (16) having a grip surface (17) is provided on the nozzle (5).

6. Fluid discharge head according to claim 5, **characterized in that** the second, additional skirt (12), which is fabricated from a different material from that of the discharge nozzle (5), is formed on the push-on part (16).

7. Fluid discharge head according to claim 6, **characterized in that** the second, addition skirt (12) consists of a softer plastic than the other skirt (9).

## Revendications

1. Tête de décharge de fluide (1) avec une tubulure de décharge (5) pouvant être placée sur un dispositif de décharge (2), qui reçoit un système de guidage de milieux et une sortie de milieux au niveau de l'extrémité du système de guidage de milieux, un élément de fermeture mobile pouvant être transféré dans une position d'ouverture étant prévu dans la zone de la sortie de milieux, et la tubulure de décharge (5) et le dispositif de décharge (2) pouvant être déplacés de manière axiale l'un par rapport à l'autre aux fins de l'actionnement de la décharge, la tubulure de décharge (5) présentant à cet effet une jupe (9) afin de former un système de guidage glissant avec une section (11) au niveau du dispositif de décharge (2), une deuxième jupe (12) extérieure supplémentaire étant associée à la jupe (9) de la tubulure de décharge (5) afin de réaliser une douille annulaire (13), qui peut être montée de manière coulissante en longueur sur une section (11), réalisée en tant que tige annulaire, du dispositif de décharge (2) et la douille annulaire (13) présentant une came (14) pour un système de sécurité anti-retrait, **caractérisée en ce que** l'élément de fermeture présente un corps de soupape (10) contraint de manière élastique, qui est réalisé en tant que piston cylindrique, qui peut être coulissé de manière axiale dans une extrémité en forme de pot formée par un manchon intérieur (18), délimitant un canal de milieux (8) du système de guidage de milieux, de la tubulure de décharge (5), et le piston présente une tête de soupape intermédiaire (25), qui étanchéifie une chambre de pression (22) raccordée au système de guidage de milieux menant à un siège de soupape (23) supérieur du corps de soupape, le système de guidage de milieux (8) s'étend en tant que canal de passage (24) à travers le corps de soupape (10) de sorte que le corps de soupape (10) soit traversé par un milieu, le corps de soupape (10) présentant à cet effet le canal de passage (24), qui relie le canal de milieux (8) à la chambre de pression (22).

2. Tête de décharge de fluide selon la revendication 1, **caractérisée en ce que** la deuxième jupe (12) supplémentaire est réalisée de manière élastique radialement.

3. Tête de décharge de fluide selon la revendication 1 ou 2, **caractérisée en ce que** la deuxième jupe (12) supplémentaire est réalisée plus courte que l'autre jupe (9) de telle manière qu'une rampe d'encliquetage radiale est prévue,

4. Tête de décharge de fluide selon l'une quelconque des revendications 1 à 3, **caractérisée en ce qu'**afin d'ouvrir le siège de soupape (23) supérieur dans la chambre de pression (22), une pression de décharge de milieux peut être réglée, laquelle est plus élevée qu'une force élastique bloquant le corps de soupape (10).

5. Tête de décharge de fluide selon l'une quelconque des revendications 1 à 4, **caractérisée en ce qu'**une partie d'emboîtement (16) avec une face de préhension (17) est prévue au niveau de la tubulure (5).

6. Tête de décharge de fluide selon la revendication 5, **caractérisée en ce que** la deuxième jupe (12) supplémentaire est réalisée au niveau de la partie d'emboîtement (16), laquelle est fabriquée à partir d'un matériau autre que la tubulure de décharge (5).

7. Tête de décharge de fluide selon la revendication 6, **caractérisée en ce que** la deuxième jupe (12) supplémentaire est constituée d'une matière plastique plus souple que celle de l'autre jupe (9).
